**Europäisches Patentamt**

**European Patent Office**

**Office européen des brevets**

(19)

(11) Veröffentlichungsnummer: **0 148 299 B2**

# (12) NEUE EUROPÄISCHE PATENTSCHRIFT

(45) Veröffentlichungstag der neuen Patentschrift:
**18.12.91 Patentblatt 91/51**

(51) Int. Cl.⁵: **C12N 1/20, A23C 9/123**

(21) Anmeldenummer: **84100192.8**

(22) Anmeldetag: **10.01.84**

(54) **Bakterielle Mischkultur, Verfahren zum Herstellen einer solchen Mischkultur, ein diese Mischkultur enthaltendes Mittel, sowie Verfahren zum Herstellen von Joghurt unter Verwendung einer solchen Mischkultur.**

(43) Veröffentlichungstag der Anmeldung:
**17.07.85 Patentblatt 85/29**

(45) Bekanntmachung des Hinweises auf die Patenterteilung:
**12.08.87 Patentblatt 87/33**

(45) Bekanntmachung des Hinweises auf die Entscheidung über den Einspruch:
**18.12.91 Patentblatt 91/51**

(84) Benannte Vertragsstaaten:
**AT BE CH FR IT LI LU NL**

(56) Entgegenhaltungen:
**DE-A- 2 455 833**

(56) Entgegenhaltungen:
**J. RASIC u.a.: "YOGHURT", Fermented Fresh Milk Products, Band 1, Seiten 50-52, 103-104, 139, 210-211, 215, 218; Technical Dairy Publishing House, Kopenhagen, DK**
**Milchwissenschaft 35(8), 1980, S. 470-473**
**Deutsche Milchwirtschaft 8(1982), S. 268-272**
**Zuivelzicht 7, 74 (1982), S. 176-178**

(73) Patentinhaber: **SANOFI BIO-INDUSTRIES GMBH**
**Kanzlerstrasse 6**
**W-4000 Düsseldorf 30 (DE)**

(72) Erfinder: **Klupsch, Hans Joachim**
**Osterflierich Pedinghauser Markt 9**
**W-4700 Hamm 1 (DE)**

(74) Vertreter: **Patentanwälte Grünecker, Kinkeldey, Stockmair & Partner**
**Maximilianstrasse 58**
**W-8000 München 22 (DE)**

EP 0 148 299 B2

## Beschreibung

Die Erfindung bezieht sich auf eine bakterielle Mischkultur, die zwei zur Joghurtherstellung geeignete Stämme enthält, ein Verfahren zum Herstellen einer solchen Mischkultur, ein Mittel zum Herstellen von Joghurt sowie ein Verfahren zum Herstellen von Joghurt.

Beim Herstellen von Joghurt wird bisher üblicherweise ein Milchprodukt, beispielsweise Vollmilch oder Magermilch mit Mischkulturen beimpft, die in der Regel zwei, unterschiedlichen Bakterienarten angehörende Stämme enthält. Stichfestes oder pastöses Joghurt mit angenehmem Aroma erhält man dann, wenn die Mischkultur neben den für das Produkt charakteristischen Stäbchenbakterien der Art Lactobacillus bulgaricus noch Bakterien der Art Streptococcus thermophilus enthält. L.bulgaricus säuert Milch sehr stark bis zu einem pH-Wert von 3,65 an und ist für das typische Joghurtaroma verantwortlich. Nachteilig an dieser Bakterienart ist jedoch, daß sie die unerwünschte D(-)-Milchsäure, also linksdrehende Milchsäure erzeugt und kein stichfestes oder pastöses Produkt, sondern lediglich ein lockeres Koagulum liefert. Die zweite in der Mischkultur vorhandene Bakterienart Sc. thermophilus säuert die Milch dagegen nur verhältnismäßig schwach an, nämlich bis zu einem pH-Wert von etwa 4,8 und produziert nicht das typische Joghurtaroma. Diese Bakterienart erzeugt jedoch die erwünschte L(+)-Milchsäure, also rechtsdrehende Milchsäure und ist dafür verantwortlich, daß der Joghurt stichfest oder pastös wird, je nach dem, wie der Joghurt verarbeitet wird. Keine der beiden, in der typischen Joghurtkultur verwendeten Bakterienarten besitzt somit für sich allein alle Stoffwechseleigenschaften, die für das angestrebte aromatische und stichfeste oder pastöse Joghurt unerläßlich sind, so daß beide Arten zusammen verwendet werden. Zugleich müssen aber auch die Nachteile beider in Kauf genommen werden. Die nachstehende Tabelle zeigt die Verteilung der vor- und nachteiligen Eigenschaften der in den Mischkulturen des Standes der Technik verwendeten Bakterienstämme.

| Bakterienart | Vorteile | Nachteile |
|---|---|---|
| L. bulgaricus | Aromabildung, schnelle Säue-rung | Bildung von Bitter-stoffen, Nachsäuerung, Bildung von D(-)-Milch-säure, kein stichfes-tes o.pastöses Produkt |
| Sc. thermophilus | stichfestes Produkt, keine Nachsäuerung, Bildung von L(+)-Milchsäure | langsame Säuerung, kein Aroma |

Da in letzter Zeit vom medizinischen Standpunkt aus in zunehmenden Maße Bedenken gegen linksdrehende Milchsäure geäußert werden, empfiehlt die Weltgesundheitsorganisation WHO, die tägliche Aufnahme von D(-)-Milchsäure auf 100 mg/kg Körpergewicht zu beschränken. Da Joghurt ein Nahrungsmittel ist, das in größtem Umfang konsumiert wird, ist es ein dringendes Anliegen, in diesem Nahrungsmittel die Menge von D(-)Milchsäure stark zu reduzieren. Es ist bis jetzt jedoch nicht gelungen, stäbchenförmige Lactobacillen herzustellen, die keine D(-)-Milchsäure enthielten. Andererseits ist die Anwesenheit von Lactobacillus bulgaricus in einem Sauermilchprodukt, das die Bezeichnung Joghurt" trägt, gesetzlich vorgeschrieben. Da Lactobacillus bulgaris zu denjenigen Bakterienarten gehört, die bei ihrer Vermehrung durch Verstoffwechselung der Lactose zu 100 % D(-)-Milchsäure produzieren, ist es im Stand der Technik bisher unvermeidbar, daß Sauermilchprodukte mit der Bezeichnung Joghurt" einen unerwünscht hohen Anteil an D(-)-Milchsäure enthalten. Das Verhältnis von unerwünschter linksdrehender zu erwünschter rechtsdrehender Milchsäure hängt somit einerseits von den verwendeten Bakterienarten ab. Von wesentlichem Einfluß auf das Verhältnis der beiden Milchsäure-Isomeren sind jedoch auch die folgenden drei weiteren Faktoren:

1. Der End-pH-Wert nach der Fermentation (Säuregrad des fertigen Produktes);
2. die Nachsäuerung bis Ablauf des Haltbarkeitsdatums bzw. bis zum Konsum des Produktes (Säuregrad des gelagerten Produktes);
3. der Einfluß des Rohstoffes Milch auf die Nachsäuerung.

Infolge des Aminosäurenkommensalismus geschieht während der Bebrütung die Vermehrung der beiden Symbionten L. bulgaricus und Sc. thermophilus in gegenseitigen Abhängigkeiten, aber so, daß die Vermehrung des L(+)-Milchsäurebildners Sc. thermophilus immer vorauseilt. Verfolgt man unter dem Mikroskop das Stäbchen: Kokken-Verhältnis während der Bebrütung, also das Verhältnis L. bulgaricus: Sc. thermophilus, dann erkennt man die Dominanz der Kokken bis zu einem pH-Bereich von etwa 4,6. Das erklärt, weshalb im frischen Joghurt der L(+)-Milchsäuregehalt überwiegt und etwa bei 70 % liegt. Säuert man dagegen den Joghurt weiter, dann steigt der D(-)-Milchsäuregehalt ständig an und vermindert den Relativanteil an L(+)-Milchsäure. Wird Joghurt bis zur möglichen Untergrenze des pH-Wertes gesäuert, dann steigt der Anteil an D(-)-Lactat auf über 50 % (Beispiel: pastöser Rührjoghurt mit pH 3,75, L(+)- : D(-)-Milchsäure 48: 52, Gesamtmilchsäure 1,2 %). Hieraus wird verständlich, daß pastöser Rührjoghurt höhere D(-)Milchsäurewerte aufweist als stichfester Joghurt, da Rührjoghurt aus technologischen Gründen tiefer gesäuert werden muß und andererseits bei stichfestem Joghurt die Säuerung bei höheren pH-Werten beendet werden kann.

Es ist weiterhin bekannt, daß während der Lagerung bis zum Konsum der D(-)-Milchsäuregehalt ansteigt. Es besteht eine Beziehung zwischen dem auf Nachsäuerung beruhenden Anstieg des Gesamt-Milchsäuregehaltes und dem Anstieg des D(-)-Milchsäuregehaltes. Die Nachsäuerung beim Joghurt dürfte demnach vor allem auf dem Anstieg des D(-)-Milchsäuregehaltes beruhen. Eine Stütze der Behauptung des Zusammenhanges zwischen Nachsäuerung und D(-)-Milchsäuregehalt ist die Feststellung, daß in Fruchtjoghurtproben mit Sorbinsäure, die das Nachsäuern verhindert, der mittlere D(-)-Milchsäuregehalt insgesamt niedriger lag (Kielwein, G., und Daun, Ursula, Vorkommen und Bedeutung von D(-)-Milchsäure in fermentierten Milcherzeugnissen unter besonderer Berücksichtigung von Milcherzeugnissen auf Joghurtbasis." Deutsche Molkerei-Zeitung, Folge 8, 1979).

Nach Kielwein und Daun (a.a.O.) steht ein hoher D(-)-Milchsäuregehalt auch im Zusammenhang mit dem bakteriologischen Status der Anlieferungsmilch sowie auch mit einer Rekontamination der Produkte mit L. bifermentans, letzteres insbesondere bei Fruchtjoghurt, wenn auch bisher der Nachweis von L. bifermentans in Fruchtjoghurt nur in wenigen Proben gelungen ist. Daß Joghurt aus keimreicher Milch besser säuert als einer aus keimarmer Milch, gehört zum Erfahrungsgut des Praktikers. Kunath und Kandler ("Der Gehalt an L( + )- und D(-)-Milchsäure in Joghurtprodukten" Milchwissenschaft 35 (8) 1980) verweisen darauf, daß im Falle einer Anreicherung von Proteinspaltprodukten in der Rohmilch, wie Peptiden und freien Amminosäuren, eine Förderung von Sc. thermophilus und somit vermehrte L(+)-Milchsäureproduktion und im Falle der Anreicherung von Ameisensäure eine solche von L. bulgaricus, und somit eine Erhöhung des D(-)Milchsäuregehaltes zu erwarten ist.

Zusammenfassend ergibt sich somit, daß der erwünschte, möglichst hohe Anteil an L(+)-Milchsäure im Joghurt einerseits durch den Einsatz von Starterkulturen erreicht wird, die überwiegend L(+)-Milchsäure bildende Mikroorganismen enthalten.

Ein unerwünschter D(-)-Milchsäuregehalt kann darüber hinaus durch möglichst milde Säuerung und Vermeidung von zu starker Nachsäuerung reduziert werden.

Im Stand der Technik wurde ein verlangsamtes Wachstum von L. bulgaricus, der die unerwünschte D(-)-Milchsäure erzeugt, dadurch erreicht, daß die Joghurtmilch vorgezogen mit Sc. thermophilus beimpft und L. bulgaricus verzögert zugegeben wurde. Die Produktion von D(-)-Milchsäure wurde dadurch reduziert bei gleichzeitiger, ausreichender Produktion der gewünschten Joghurtaromastoffe. Es ist auch bereits auf die Möglichkeit der getrennten Beimpfung von Milchprodukten mit Sc. thermophilus und L. bulgaricus hingewiesen worden sowie auf die Züchtung des L. bulgaricus unter Bedingungen, die dessen Wachstum verlangsamen. Dazu gehörten Maßnahmen wie Senkung der Bebrütungstemperatur sowie Reduzierung der Bebrütungszeit.

Diese genannten Verfahren sind jedoch unter den heutigen großtechnischen Produktionsbedingungen nicht zu verwirklichen, da beim Einsatz der genannten Verfahrensweisen bei der großtechnischen Produktion tatsächlich der L( + )-Milchsäuregehalt nicht wesentlich erhöht wird.

Der Erfindung liegt die Aufgabe zugrunde, eine bakterielle Mischkultur, die zwei zur Joghurtherstellung geeignete Stämme enthält, zur Verfügung zu stellen, durch die gewährleistet ist, daß unter großtechnischen Produktionsbedingungen der Gehalt von physiologischer L(+)-Milchsäure zu über 70 % im Endprodukt auch noch nach der üblichen Haltbarkeitsgarantie vorhanden ist und das Produkt während der Lagerzeit nur so wenig nachsäuert, daß auch nach Ablauf der Haltbarkeitsgarantie der pH-Wert nicht tiefer als etwa pH 4,4 bis 4,3 liegt, was dem pH-Wert eines frischerzeugten Joghurts nach der herkömmlichen Methode entspricht.

Diese Aufgabe wird, ausgehend von einer bakteriellen Mischkultur laut Oberbegriff des Hauptanspruchs, dadurch gelöst, daß sie auf die Weise hergestellt wird, daß ein Milchausgangsmaterial als Nährsubstrat zuerst mit einer Kultur eines allgemein erhältlichen Stammes von Sc. thermophilus in einem Verhältnis von etwa 1 bis 10 %, vorzugsweise 2 bis 6 %, beimpft und bei geeigneter Temperatur bis zum Erreichens eines pH-Wertes von etwa 4,0 bis 4,7, vorzugsweise von etwa 4,4, bebrütet wird und danach das Milchausgangsmaterial mit einer Kultur eines allgemein erhältlichen Stammes von L. bulgaricus beimpft wird wobei das Verhältnis Streptoccus thermophilus zu Lactobacillus bulgarius etwa 1000 : 1 bis 100:1 beträgt und die Mischkultur sodann auf übliche Weise konserviert wird.

Mit dieser erfindungsgemäßen Mischkultur wird erstmals eine Impfkultur zum Herstellen von Joghurt zur Verfügung gestellt, die zwar einerseits die gesetzlich vorgeschriebene Mikroflora, bestehend aus L. bulgaricus und Sc. thermophilus, enthält und dennoch bei der großtechnischen Produktion der besonders unerwünschte Nachteil einer erhöhten D(-)-Milchsäureherstellung vermieden wird.

Der Effekt der erfindungsgemäßen, durch das Verfahren zu seiner Herstellung definierten Mischkultur, nämlich in dem zu säuernden Produkt nie mehr als maximal 30 % D(-)-Milchsäure zu produzieren, war insofern völlig überraschend, als es zwar verständlich ist, daß wenig D(-)-Milchsäure produziert wird, wenn das Endprodukt mit dem Stamm L. bulgaricus nachbeimpft wird, es aber keineswegs auf der Hand lag, daß der gewünschte Effekt auch eintritt, wenn beim Herstellen der sogenannten Starterkultur der linksdrehende Milchsäure produzierende Stamm L. bulgaricus erst nach einer gewissen Wachstumsdauer von Sc. thermophilus nachbeimpft wird. In Kenntnis der Literaturstelle Kunath und Kandler (a.a.O.), in der ausgeführt wurde, daß zwar in der Regel in den Joghurtprodukten aus dem Handel ein Überschuß an L(+)-Milchsäure gefunden wurde, sich jedoch bei denjenigen Produkten, die eine gemischte Streptokokken- und Stäbchenflora enthalten, zum Zeitpunkt des Verkaufs bzw. Verzehrs kein weit überwiegender L(+)-Milchsäuregehalt von 70 % und mehr garantieren ließe, stand dem Fachmann vielmehr ein massives Vorurteil entgegen, das mit der vorliegenden Erfindung beseitigt wurde. Es war insbesondere völlig unerwartet, daß die genannten Probleme durch das zur Verfügung stellen einer einzigen Starterkultur, die entsprechend leicht gehandhabt werden kann, gelöst werden könnten.

Mit der erfindungsgemäßen Starterkultur, die durch den product-by-process-Anspruch definiert ist, ist es somit erstmals gelungen, definitionsgemäßen Joghurt, also ein Sauermilchprodukt, das die Stämme L. bulgaricus und Sc. thermophilus enthält, großtechnisch herzustellen und trotzdem im Endprodukt einen Gehalt an physiologischer L(+)-Milchsäure zu garantieren, der mindestens über 70 % liegt. Diese Tatsache wurde in einer Blinduntersuchung im Institut für Mikrobiologie der Universität Bonn, Labor für Lebensmittel-Mikrobiologie bestätigt.

Bei dieser Untersuchung wurde die Konzentration beider Milchsäureisomeren in drei verschiedenen Sauermilchprodukten bestimmt. Die Proben erhielten die Bezeichnungen Y1, Y2 und Y3.

Bei der Probe Y1 handelte es sich um eine Laborprobe von Bifighurt®, das den Stamm Bifidobakterium bifidus enthält, der ein Racemat aus sowohl D(-)- als auch L(+)-Milchsäure bildet, jedoch L(+)-Milchsäure im Überschuß von 95 %. Als Probe Y2 wurde eine Joghurtprobe untersucht, die unter Verwendung der erfindungsgemäßen bakteriellen Mischkultur hergestellt wurde. Die Probe Y3 enthielt Bioghurt® aus der laufenden Herstellung, der die beiden Stämme B. bifidus und L. acidophilus enthält. Beide Stämme produzieren weit überwiegend L(+)-Milchsäure.

Die Untersuchung ergab das in Tabelle I gezeigte Ergebnis.


**Tabelle I**

| Proben | L(+)-Milchsäure mg /100 g (%) | D(-)-Milchsäure mg/100 g (%) |
|---|---|---|
| Y1 | 560 (95) | 24 (5) |
| Y2 | 663 (90) | 60 (10) |
| Y3 | 655 (97) | 15 (3) |


Bei unterschiedlichem Gesamtmilchsäuregehalt und einem pH-Wert von etwa 4,3 wurde somit bei den drei untersuchten Proben ein hoher, ernährungsphysiologisch wertvoller L(+)-Milchsäureanteil von mehr als 90 % festgestellt. Es zeigte sich, daß die Probe Y2, also das mit der erfindungsgemäßen bakteriellen Mischkultur hergestellte Joghurt in seinem Gehalt an L(+)-Milchsäure kaum solchen Sauermilchprodukten nachsteht, die mit Bakterienstämmen beimpft wurden, die ausschließlich oder nahezu ausschließlich L(+)-Milchsäure produzieren.

Die untersuchten Proben waren alle geruchlich einwandfrei, besaßen einen sauren, abgerundet vollmundigen Geschmack und waren von guter Konsistenz. Das heißt, daß auch die Probe Y2 den erwarteten, von L. bulgaricus produzierten Joghurtgeschmack aufwies. Dennoch ist durch den Einsatz der erfindungsgemäßen bakteriellen Mischkultur der Gehalt an unerwünschter linksdrehender Milchsäure auf ein Mindestmaß reduziert.

Die Produktion von L(+)-Milchsäure ist dann mit besonderer Sicherheit gewährleistet, wenn das Bebrüten des Sc. thermophilus bei etwa 42°C während einer Dauer von etwa 4 bis 6 Stunden in dem als Nährsubstrat geeigneten Milchausgangsmaterial durchgeführt wird und sodann mit dem Stamm L. bulgaricus beimpft wird.

Um die eingangs erwähnten Einflüsse des Rohstoffes Milch auf die Nachsäuerung möglichst weitgehend kontrollieren zu können, ist es zweckmäßig, das Milchausgangsmaterial vor dem Beimpfen auf etwa 95°C zu erhitzen und danach auf etwa 42°C abzukühlen, ehe mit dem Stamm Sc. thermophilus beimpft wird.

Um das Wachstum der eingesetzten Stämme bei der Herstellung der erfindungsgemäßen Bakterienmischkultur zu fördern, kann es sich als günstig erweisen, dem Milchausgangsmaterial zusätzliches Nährsubstrat zuzufügen. Wenn das Mischungsverhältnis von Sc. thermophilus und L.bulgaricus etwa 1000:1 bis 100:1 beträgt, ist in optimaler Weise ein Kompromiß verwirklicht, mit dem der Gehalt an unerwünschter linksdrehender Milchsäure reduziert, andererseits aber auch ausreichend Aromastoffe für den typischen Joghurtgeschmack produziert werden.

Die erfindungsgemäße bakterielle Mischkultur gemäß einem der Ansprüche 1 bis 5, kann auf übliche Weise konserviert werden, also entweder als lyophilisierte Trockenkultur, als tiefgefrostetes Kulturkonzentrat, als Flüssigkultur oder Flüssigkulturkonzentrat aufbewahrt werden.

Die im Handel frei erhältlichen Ausgangskulturen von Sc.thermophilus und L. bulgaricus, mit denen das Milchausgangsmaterial erfindungsgemäß beimpft wird, weisen eine Zellzahl von je etwa $10^8$-$10^{12}$ pro ml auf.

Neben der bakteriellen Mischkultur bezieht sich die Erfindung auch auf ein Verfahren zum Herstellen dieser bakteriellen Mischkultur, die zwei zur Joghurtherstellung geeignete Stämme enthält. Wie bereits erläutert, liegt das Neue und Erfinderische der beanspruchten bakteriellen Mischkultur in den neuen und erfinderischen Verfahrensschritten zu seiner Herstellen. Diese wurden bereits erläutert.

In der bakteriellen Mischkultur gemäß den Ansprüchen 1 bis 5 wird in erster Linie ein Mittel zum Herstellen von Joghurt zur Verfügung gestellt, das die in den Ansprüchen 11 bis 15 beschriebenen Vorteile und Eigenschaften aufweist, die oben im Zusammenhang mit den Ansprüchen 1 bis 5 diskutiert wurden.

Die vorliegende Erfindung bezieht sich desweiteren auf ein Verfahren zum Herstellen von Joghurt.

Die eingangs geschilderten Probleme bezüglich eines zu hohen Anteils an unerwünschter linksdrehender Milchsäure in Sauermilchprodukten des Typs Joghurt", die gesetzlich vorgeschrieben die Stämme Sc. thermophilus und L. bulgaricus enthalten müssen, werden erfindungsgemäß dadurch gelöst, daß ein Milchprodukt mit einer bakteriellen Mischkultur nach einem der Ansprüche 1 bis 5 beimpft und auf übliche Weise bebrütet wird.

Bei der Behandlung üblicher Milchprodukte mit einer bakteriellen Mischkultur gemäß den Ansprüchen 1 bis 5, beziehungsweise einer bakteriellen Mischkultur, die durch das in den Ansprüchen 6 bis 10 beschriebene Verfahren hergestellt wurde, beziehungsweise mit einem Mittel zum Herstellung von Joghurt gemäß einem der Ansprüche 11 bis 15, werden die oben ausführlich dargelegten Vorteile erzielt, nämlich ein Kompromiß zwichen dem Zurückdrängen der unerwünschten linksdrehenden Milchsäure im Endprodukt bei gleichzeitigem Erhalten des erwünschten Joghurtaromas unter großtechnischen Produktionsbedingungen.

Für ein möglichst gelichbleibendes und gut kontrollierbares Ergebnis bezüglich prozentualer Anteile der beiden Milchsäureisomeren im Endprodukt, empfiehlt es sich, ähnlich wie bei der Herstellung der bakteriellen Mischkultur, auch bei der Herstellung des Joghurts, daß das Milchprodukt vor dem Beimpfen einer üblichen Hitzebehandlung unterzogen und anschließend auf etwa 44 bis 46°C abgekühlt wird. Durch diese Maßnahme wird die Keimzahl in dem zu beimpfenden Milchprodukt reduziert, so daß die oben gescheilderten Einflüsse auf die Produktion von rechts- oder linksdrehender Milchsäure weitgehend egalisiert und beherrscht werden.

Soll ein stichfestes Joghurtprodukt erhalten werden, ist es zweckmäßig, daß das Milchprodukt mit 1 bis 10 %, vorzugsweise 3 bis 5 °%, einer Mischkultur gemäß einem der Ansprüche 1 bis 15 beimpft, in verkaufsfertige Becher abgefüllt und anschließend bei ca. 42°C bebrütet wird. Ein auf diese Weise hergestellter stichfester Joghurt ist mildsauer und weist auch nach der üblichen Haltbarkeitsgarantie von 31/2 Wochen noch einen Gehalt an phsysiologischer L(+)-Milchsäure von mit Sicherheit über 70 % auf. Das Produkt säuert auch während der Lagerzeit nur so wenig nach, daß acuh nach Ablauf der Haltbarkeitsgarantie der pH-Wert nich tiefer als pH 4,4 bis 4,3 liegt. Dies enspricht dem pH-Wert eines frischerzeugten Joghurts, das nach den Methoden des Standes der Technik hergestellt wurde.

Eine besonders bevorzugte Maßnahme, um die Nachsäuerung der bereits beimpften und in verkaufsfertige Becher abgefüllten Milch unter Kontrolle zu halten, besteht darin, daß nach Erreichen eines pH-Wertes von etwa 4,75 die Säuerung durch Kühlen unterbrochen wird. Da, wie oben bereits dargelegt, das Nachsäuern überwiegend der Fermentation von Lactose durch L. bulgaricus zuzuschreiben ist, wird durch eine kontrollierte Nachsäuerung der Gehalt an unerwünschter D(-)-Milchsäure gering gehalten. Die gewünschten Joghurtaromastoffe werden bei den geschilderten Produktionsbedingungen jedoch noch ausreichend produziert.

Auch pastöser, gerührter Joghurt mit einem minimalen Gehalt an unerwünschter D(-)-Milchsäure läßt sich unter Einsatz der erfindungsgemäßen bakteriellen Mischkultur in optimaler Weise dadurch herstellen, daß das Milchprodukt in Reifetanks auf etwa 32 bis 35°C temperiert, danach mit etwa 0,5 bis 2 % einer bakteriellen Mischkultur gemäß einen der Ansprüche 1 bis 15 beimpft, während einer Dauer von etwa 12 bis 20 Stunden bei etwa 32 bis 35°C bebrütet und nach Erreichen eines pH-Wertes von etwa 4,0 bis 4,4 in verkaufsfertige Einheiten abgefüllt wird.

Im Gegensatz zum stichfesten Joghurt wird bei dieser Produktionsweise die Säuerung in Reifetanks durchgeführt und erst das fertiggesäuerte Produkt in verkaufsfertige Einheiten abgefüllt. Üblicherweise weist gerührtes Joghurt höhere D(-)-Milchsäurewerte auf als stichfestes Joghurt, da Rührjoghurt aus technologischen Gründen tiefer gesäuert werden muß als stichfestes Joghurt. Dieser Nachteil läßt sich mit dem erfindungsgemäßen Verfahren kompensieren, indem nur mit etwa 0,5 bis 2 % einer erfindungsgemäßen bakteriellen Mischkultur das Milchprodukt in den Reifetanks beimpft wird und dann etwa 12 bis 20 Stunden bei der verhältnismäßig niedrigen Temperatur von 32 bis 35°C bebrütet wird. Unter diesen Verfahrensbedingungen läßt sich der Gehalt an D(-)-Milchsäure auch beim Rührjoghurt auf einen maximalen Anteil von etwa 30 % zurückdrängen.

Vorzugsweise erfolgt die Bebrütung während einer Dauer von 14 bis 18 Stunden bei 33° C.

Das zu beimpfende Milchprodukt kann Sahne, Vollmilch, fettarme Milch oder Magermilch sein. Je höher der Fettgehalt des Milchproduktes ist, desto höher kann der pH-Wert liegen, das heißt bei normal fetter Milch wird bis zu einem pH-Wert von 4,4, bei fettarmer Milch von 4,2 und bei Magermilch von 4,0 bebrütet.

Nach Belieben kann dem Milchprodukt eine Fruchtzubereitung zugemischt werden. Im Falle des stichfesten Joghurts wird vor dem Einfüllen des beimpften Milchproduktes in die verkaufsfertige Verpackung eine beliebige Fruchtzubereitung in die Becher vordosiert und darüber die Milch gefüllt.

Bei der Fruchtzumischung beim pastösen Rührjoghurt wird die Fruchtzubereitung nach dem Erreichen des gewünschten Säuerungsgrades zugemischt und sodann das Fruchtjoghurt in verkaufsfertige Einheiten abgefüllt.

Im folgenden wird die vorliegende Erfindung in Beispielen dargestellt.

### Beispiel I

Herstellen der erfindungsgemäßen bakteriellen Mischkultur.

Ein Milchprodukt mit gewünschtem Fettgehalt, also Sahne, Vollmilch, fettarme Milch oder Magermilch wird, falls gewünscht auch unter Verwendung eines wachstumfördernden Nährsubstrats, auf 95° C erhitzt, danach auf 43° C abgekühlt und mit einem Kulturkonzentrat eines allgemein erhältlichen Sc. thermophilus, das eine Konzentration von etwa $10^{10}$ Zellen/ml aufweist, beimpft. Das Bebrüten erfolgt bei 42° C während einer Dauer von 4 bis 6 Stunden bis pH-Wert von 4,4 erreicht ist. Dieser Kultur von Sc. thermophilus wird ein allgemein erhältliches Kulturkonzentrat von L. bulgaricus zu Sc. thermophilus von etwa $10^{10}$ Zellen/ml aufweist, in einer Menge zugemischt, daß Verhältnis von L. bugaricus zu Sc. thermophilus etwa 1:1000 ist. Diese Mischung wird in für den Handel geeignete Behälter abgefüllt und ist 5 Tage einsatzfähig.

### Beispiel II

Herstellen eines stichfesten Joghurtproduktes.

Sahne, deren Fettgehalt nach Wunsch durch Milchpulverzusatz in der Trockenmasse oder durch Eindampfen in der Trockenmasse erhöht wird, Vollmilch, fettarme Milch oder Magermilch wird der üblichen Hitzebehandlung unterzogen, auf 44 bis 46°C abgekühlt und mit 3 bis 5 % einer erfindungsgemäßen bakteriellen Mischkultur, die gemäß Beispiel I hergestellt wurde, beimpft. Die beimpfte Milch wird in verkaufsfertige Becher abgefüllt und wie üblich in einer Brutkammer oder einem Brutraum bei 42°C bebrütet. Wenn ein pH-Wert von etwa 4,75 erreicht ist, wird die Säuerung durch Kühlen unterbrochen. Die Lagerung bis zum Verkauf erfolgt wie üblich bei +4°C. Ein auf diese Weise großtechnisch hergestelltes Joghurt enthält mindestens 70 %, optimalerweise jedoch 90 %, der physiologisch erwünschten L(+)-Milchsäure und weist einen angenehmen, abgerundeten Joghurtgeschmack auf.

### Beispiel II

Herstellen eines stichfesten Fruchtjoghurts.

Die Milchvorbehandlung und das Beimpfen geschieht wie im Beispiel II geschildert. Vor dem Einfüllen in die verkaufsfertige Verpackung wird eine beliebige Fruchtzubereitung in die Becher vordosiert und darüber die Milch gefüllt. Das Bebrüten und Abkühlen erfolgt wie in Beispiel II beschrieben.

### Beispiel IV

Herstellen von pastösem Rührjoghurt.

Die wie in Beispiel II beschriebene vorbehandelte Milch wird in Reifetanks auf 33°C temperiert und mit 0,5 bis 2,0 % einer erfindungsgemäßen bakteriellen Mischkultur, deren Herstellung in Beispiel I beschrieben wurde, beimpft. Das Bebrüten erfolgt während einer Dauer von 14 bis 18 Stunden bei 33°C bis ein pH-Wert von 4,4 bei Sahne oder Vollmilch, von 4,2 bei fettarmer Milch oder 4,0 bei Magermilch erreicht ist. Danach wird das gesäuerte Milchprodukt in den Reifetanks gekühlt und sodann in verkaufsfertige Einheiten abgefüllt. Das Produkt wird bis zum Verkauf kühlgehalten.

**Beispiel V**

Herstellen von pastösem Rührjoghurt mit Fruchtzusatz.

Es wird verfahren wie in Beispiel IV, jedoch wird nach dem ausreichenden Säuern und Abkühlen des in den Reifetanks befindlichen Milchproduktes die gewünschte Fruchtzubereitung zugemischt. Danach erfolgt das Abfüllen in verkaufsfertige Einheiten und anschließendes Kühlen bis zum Verkauf.

Beim Einsatz der erfindungsgemäßen bakteriellen Mischkultur und Durchführen des erfindungsgemäßen Verfahrens zum Herstellen von Joghurt liegt der Gehalt an physiologicher L(+)-Milchsäure bei mindestens 70 % regelmäßig jedoch bei etwa 90 % und dies bei Produkten, die bereits über die übliche Haltbarkeitsgrenze von 31 /2 Wochen gelagert waren. Dennoch weisen die Joghurtprodukte das gewünschte Aroma auf.

**Patentansprüche**

1. Bakterielle Mischkultur, die zwei zur Joghurtherstellung geeignete Stämme enthält, dadurch <u>gekennzeichnet</u>, daß sie auf die Weise hergestellt wird, daß ein Milchausgangsmaterial als Nährsubstrat zuerst mit einer Kultur eines allgemein erhältlichen Stammes von Streptococcus thermophilus in einem Verhältnis von etwa 1 bis 10 %, vorzugsweise 2 bis 6 %, beimpft und bei geeigneter Temperatur bis zum Erreichen eines pH-Wertes von etwa 4,0 bis 4,7, vorzugsweise von etwa 4,4, bebrütet wird und danach das Milchausgangsmaterial mit einer Kultur eines allgemein erhältlichen Stammes von Lactobacillus bulgaricus beimpft wird wobei das Verhältnis von Streptococcus thermophilus zu Lactobacillus bulgaricus etwa 1000:1 bis 100:1 beträgt und die Mischkultur sodann auf übliche Weise konserviert wird.

2. Bakterielle Mischkultur nach Anspruch 1, dadurch <u>gekennzeichnet</u>, daß das Bebrüten des Stammes Streptococcus thermophilus bei etwa 42°C während einer Dauer von etwa 4 bis 6 Studen durchgeführt wird.

3. Bakterielle Mischkultur nach Anspruch 1 und/oder Anspruch 2, dadurch <u>gekennzeichnet</u>, daß das Milchausgangsmaterial vor dem Beimpfen auf etwa 95°C erhitzt und danach auf etwa 42°C abgekühlt wird.

4. Bakterielle Mischkultur nach mindestens einem der vorhergehenden Ansprüche, dadurch <u>gekennzeichnet</u>, daß dem Milchausgangsmaterial Nährsubstrat zugefügt wird.

5. Verfahren zum Herstellen einer bakteriellen Mischkultur, die zwei zur Joghurtherstellung geeignete Stämme enthält, dadurch <u>gekennzeichnet</u>, daß ein Milchausgangsmaterial als Nährsubstrat zuerst mit einer Kultur eines allgemein erhältlichen Stammes von Streptococcus thermophilus in einem Verhältnis von etwa 1 bis 10 %, vorzugsweise 2 bis 6 %, beimpft und bei geeigneter Temperatur bis zum Erreichen eines pH-Wertes von etwa 4,0 bis 4,7, vorzugswise von etwa 4,4, betrütet wird und danach das Milchausgangsmaterial mit einer Kultur eines allgemein erhältlichen Stammes von Lactobacillus bulgaricus beimpft wird wobei das Verhältnis von Streptococcus thermophilus zu Lactobacillus bulgaricus etwa 1000:1 bis 100:1 beträgt und die Mischkultur sodann auf übliche Weise konserviert wird.

6. Verfahren zum Herstellen einer bakteriellen Mischkultur nach Anspruch 5, dadurch <u>gekennzeichnet</u>, daß das Bebrüten des Stammes Streptococcus thermophilus bei etwa 42°C während einer Dauer von etwa 4 bis 6 Stunden durchgeführt wird.

7. Verfahren zum Herstellen einer bakteriellen Mischkultur nach Anspruch 5 und/oder Anspruch 6, dadurch <u>gekennzeichnet</u>, daß das Milchausgangsmaterial vor dem Beimpfen auf etwa 95°C erhitzt und danach auf etwa 42°C abgekühlt wird.

8. Verfahren zum Herstellen einer bakteriellen Mischkultur nach mindestens einem der Ansprüche 5 bis 7, dadurch <u>gekennzeichnet</u>, daß dem Milchausgangsmaterial Nährsubstrat zugefügt wird.

9. Mittel zum Herstellen von Joghurt, dadurch <u>gekennzeichnet</u>, daß es auf die Weise hergestellt wird, daß ein Milchausgangsmaterial als Nährsubstrat zuerst mit einer Kultur eines allgemein erhältlichen Stammes von Streptococcus thermophilus in einem Verhältnis von etwa 1 bis 10 %, vorzugsweise 2 bis 6 %, beimpft und bei geeigneter Temperatur bis zum Erreichen eines pH-Wertes von etwa 4,0 bis 4,7 vorzugsweise von etwa 4,4, betrütet wird und danach das Milchausgangsmaterial mit einer Kultur eines allgemein erhältlichen Stammes von Lactobacillus bulgaricus beimpft wird wobei das Verhältnis von Streptococcus thermophilus zu Lactobacillus bulgaricus etwa 1000:1 bis 100:1 beträgt und die Mischkultur sodann auf übliche Weise konserviert wird.

10. Mittel zum Herstellen von Joghurt nach Anspruch 9, dadurch <u>gekennzeichnet</u>, daß das Bebrüten des Stammes Streptococcus thermophilus bei etwa 42°C während einer Dauer von etwa 4 bis 6 Stunden durchgeführt wird.

11. Mittel zum Herstellen von Joghurt nach Anspruch 9 und/oder Anspruch 10, dadurch <u>gekennzeichnet</u>, daß das Milchausgangsmaterial vor dem Beimpfen auf etwa 95°C erhitzt und danach auf etwa 42°C abgekühlt wird.

12. Mittel zum Herstellen von Joghurt nach mindestens einem der Ansprüche 9 bis 11, dadurch <u>gekenn-</u>

EP 0 148 299 B2

zeichnet, daß Milchausgangsmaterial Nährsubstrat zugefügt wird.

13. Verfahren zum Herstellen von Joghurt, dadurch gekennzeichnet, daß ein Milchprodukt mit einer bakteriellen Mischkultur gemäß einem der Ansprüche 1 bis 12 beimpft und auf übliche Weise bedrütet wird.

14. Verfahren nach Anspruch 13 dadurch gekennzeichnet, daß das Milchprodukt vor dem Beimpfen einer üblichen Hitzebehandlung unterzogen und anschließend auf etwa 44 bis 46°C abgekühlt wird.

15. Verfahren nach Anspruch 13 und/oder Anspruch 14, dadurch gekennzeichnet, daß das Milchprodukt mit 1 bis 10 %, vorzugsweise 3 bis 5 %, einer Mischkultur gemäß einem der Ansprüche 1 bis 15 beimpft, in verkaufsfertige Becher abgefüllt und anschließend bei 42°C bebrütet wird.

16. Verfahren nach mindestens einem der Ansprüche 13 bis 15, dadurch gekennzeichnet, daß bei Erreichen eines pH-Wertes von etwa 4,75 die Säuerung durch Kühlen unterbrochen wird.

17. Verfahren nach Anspruch 13 und/oder 14, dadurch gekennzeichnet, daß das Milchprodukt in Reifetanks auf etwa 32 bis 35°C temperiert, danach mit etwa 0,5 bis 2 % einer Mischkultur nach mindestens einem der Ansprüche 1 bis 5 beimpft, während einer Dauer von etwa 12 bis 20 Stunden bei etwa 32 bis 35°C bebrütet und nach dem Erreichen eines pH-Wertes von etwa 4,0 bis 4,4 in verkaufsfertige Einheiten abgefüllt wird.

18. Verfahren nach mindestens einem der Ansprüche 13 bis 18, dadurch gekennzeichnet, daß das Milchprodukt Sahne oder Vollmilch oder fettarme Milch oder Magermilch ist.

19. Verfahren nach mindestens einem der Ansprüche 13 bis 19, dadurch gekennzeichnet, daß eine Fruchtzubereitung zugemischt wird.

## Claims

1. Mixed bacterial culture containing two strains suitable for voghurt manufacture, characterized in that it is produced by initially inoculating a lactic starting material as nutrient substrate wich a culture of a generally available strain of Streptococcus thermophilus at a ratio of approximately 1 to 10% preferably 2 to 6% and by incubation at a suitable temperature until a pH value of approximately 4.0 to 4.7, preferably approximately 4.4, is reached, and thereafter inoculating the lactic starting material with a culture or a generally available strain or Lactobacillus bulgaricus, whereby the ratio of Streptococcus thermophilus to Lactobacillus bulgaricus is approximatively 1000:1 to 100:1, and then conserving the mixed culture in a conventional manner.

2. Mixed bacterial culture according to claim 1, **characterised in that** incubation of the Streptococcus thermophilus strain is carried out at approximately 42°C for a period or approximately 4 to 6 hours.

3. Mixed bacterial culture according to claim 1 and/or claim 2, **characterised in that** the lactic starting material is heated to approximately 95°C and subsequently cooled to approximately 42°C prior to inoculation.

4. Mixed bacterial culture according to at least one of the above claims, **characterised in that** nutrient substrate is added to the lactic starting material.

5. Method for the manufacture of a mixed bacterial culture containing two strains suitable for the manufacture of yoghurt, **characterised in that** a lactic starting material as nutrient substrate is initially inoculated with a culture of a generally available strain of Streptococcus thermophilus at a ratio of approximately 1 to 10%, preferably 2 to 6%, and then incubated at a suitable temperature until a pH value of approximately 4.0 to 4.7, preferably 4.4, is reached, and thereafter the lactic starting material is inoculated with a culture of a generally available strain of Lactobacillus bulgaricus, whereby the ratio of Streptococcus thermophilus and Lactobacillus bulgaricus is approximately 1000:1 to 100:1, and the mixed culture is then conserved in a conventional manner.

6. Method for the manufacture of a mixed bacterial culture according to claim 5, **characterised in that** the incubation of the strain Streptococcus thermophilus is carried out at approximately 42°C for a period of approximately 4 to 6 hours.

7. Method for the manufacture of a mixed bacterial culture according to claim 5 and/or claim 6, **characterised in that** the lactic starting material is heated to approximately 95°C and then cooled to approximately 42°C prior to inoculation.

8. Method for the manufacture of a mixed bacterial culture according to at least one of the claims 5 to 7, **characterised in that** nutrient substrate is added to the lactic starting material.

9. Composition for the manufacture of yoghurt, **characterised in that** it is produced by initially inoculating a lactic starting material as nutrient substrate with a culture of a generally available strain of Streptococcus thermophilus at a ratio or approximately 1 to 10%, preferably 2 to 6%, and incubation at a suitable temperature until reaching a pH value of approximately 4.0 to 4.7, preferably approximately 4.4, and thereafter inoculating the lactic starting material with a culture of a generally available strain of Lactobacillus bulgaricus, whereby the ratio of Streptococcus thermophilus to Lactobacillus bulgaricus is approximately 1000:1 to 100:1, and the mixed culture is then conserved in a conventional manner.

10. Composition for the manufacture of yoghurt according to claim 9, **characterised in that** the incubation

8

of the strain Streptococcus thermophilus is carried out at approximately 42°C for a period of approximately 4 to 6 hours.

11. Composition for the manufacture of yoghurt according to claim 9 and/or claim 10, **characterised in that** the lactic starting material is heated to approximately 95°C and then cooled to approximately 42°C prior to inoculation.

12. Composition for the manufacture of yoghurt according to at least one of the claims 9 to 11, **characterised in that** nutrient substrate is added to the lactic starting material.

13. Method for the manufacture of yoghurt, **characterised in that** a lactic product is inoculated with a mixed bacterial culture according to one of the claims 1 to 12 and incubated in a conventional manner.

14. Method according to claim 13, **characterised in** that the lactic product is heat treated in the usual manner and then cooled to approximately 44 to 46°C prior to inoculation.

15. Method according to claim 13 and/or 14, **characterised in that** the lactic product is inoculated with 1 to 10%, preferably 3 to 5%, of a mixed culture according to one of the claims 1 to 2 filled into vending cartons and then incubated at 42°C.

16. Method according to at least one of the claims 13 to 15, **characterised in that** acidification is interrupted by way of cooling on reaching a pH value of approximately 4.75.

17. Method according to claim 13 and/or 14, **characterised in that** the lactic product is brought to a temperature of approximately 32 to 35°C in maturing tanks, then inoculated with approximately 0.5 to 2% of a mixed culture according to at least one of the claims 1 to 4 incubated for a period of approximately 12 to 20 hours at approximately 32 to 35°C, and filled into vending containers after having reached a pH value of approximately 4.0 to 4.4.

18. Method according to at least one of the claims 13 to 17, **characterised in that** the lactic product is cream, full-fat milk or semi-skimmed milk or skimmed milk.

19. Method according to at least one of the claims 13 to 18, **characterised in that** a fruit preparation is added.


**Revendications**

1. Culture bactérienne mixte contenant deux couches convenant pour la préparation du yaourt, culture caractérisée en ce qu'on l'a préparée en ensemençant une matière lactée de départ, servant de substrat nutritif, d'abord avec une culture d'une souche de Sc. thermophilus courante, dans un rapport d'environ 1 à 10 % ; de préférence de 2 à 6 %, en faisant incuber à une tempéruture appropriée jusqu'à ce que le pH atteigne une valeur d'environ 4,0 à 4,7, de préférence d'environ 4,4, puis en ensemençant la matière lactée de départ avec une culture d'une souche courante de L. bulgaricus, le rapport de Streptococcus thermophilus à Lactobacillus bulgaricus étant d'environ 1000 : 1 à 100 : 1, et en conservant ensuite la culture mixte de la manière habituelle.

2. Culture bactérienne mixte selon la revendication 1 caractérisée en ce que l'incubation de la souche Streptococcus thermophilus est effectuée à environ 42°C pendant une durée d'environ 4 à 6h.

3. Culture bactérienne mixte selon une au moins des revendications 1 et 2, caractérisée en ce que la matiére lactée de départ est chauffée à environ 95°C avant d'être ensemencée, puis est refroidie à environ 42°C.

4. Culture bactérienne mixte selon au moins une des revendications précédentes, caractérisée en ce qu'on ajoute un substrat nutritif à la matière Lactée de départ.

5. Procédé pour préparer une culture bactérienne mixte contenant deux souches appropriées pour la préparation du yaourt, procédé caractérisé en ce qu'on ensemence une matière lactée de départ, servant de substrat nutritif, d'abord avec une culture d'une souche de Streptococcus thermorphilus courante, dans un rapport d'environ 1 à 10 %, de préférence de 2 à 6 %, on fait incuber à une température appropriée jusqu'à ce que le pH atteigne uen valeur d'environ 4,0 à 4,7, de préférence d'environ 4,4, puis on ensemence la matière lactée de départ avec une culture d'une souche courante de Lactobacillus bulgaricus, le rapport de Streptococcus thermophilus à Lactobacillus bulgaricus étant d'environ 1000 : 1 à 100 : 1 et on conserve ensuite la culture mixte de la manière habituelle.

6. Procédé de préparation d'une culture bactérienne mixte selon la revendication 5, caractérisé en ce qu'on fait incuber la souche Streptotoccus thermophilus à environ 42°C pendant une durée d'environ 4 à 6 h.

7. Procédé de préparation d'une culture bactérienne mixte selon la revendication 5 et/ou la revendication 6, caractérisé en ce qu'on chauffe la matière lactée de départ à environ 95°C avant de l'ensemencer, et ensuite on la refroidit à environ 42°C.

8. Procédé de préparation d'une culture bactérienne mixte selon l'une au moins des revendications 5 à 7, caractérisé en ce qu'on ajoute un substrat nutritif à la matière lactée de départ.

9. Produit pour la préparation du yaourt, produit <u>caractérisé</u> en ce qu'on l'a préparé en ensemençant une matière lactée de départ, servant de substrat nutritif, d'abord avec une culture d'une souche de Sc. thermophilus courante, dans un rapport d'environ 1 à 10 %, de préférence de 2 à 6 %, en faisant intuber à une température appropriée jusqu'à ce que le pH atteigne uen valeur d'environ 4,0 à 4,7, de préférence d'environ 4,4, puis en ensemençant la matière lactée de départ avec une culture d'une souche courante de L. bulgaricus, le rapport de Streptococcus thermophilus à Lactobacillus bulgaricus étant d'environ 1000 : 1 à 100 : 1, et en conservant ensuite la culture mixte de la manière habituelle.

10. Produit pour la préparation du yaourt selon la revendication 9, produit <u>caractérisé</u> en ce que l'incubation de la souche Streptococcus thermophilus a été exécutée à environ 42°C pendant une durée d'environ 4 à 6 h.

11. Produit pour la préparation du yaourt selon la revendication 9 et/ou la revendication 10, produit <u>caractérisé</u> en ce que la matière lactée de départ a été chauffée à environ 95°C avant d'être ensemencée, puis a ensuite été refroidie à environ 42°C.

12. Produit pour la préparation du yaourt selon l'une au moins des revendications 9 à 11, produit <u>caractérisé</u> en ce que la matière lactée de départ a été additionnée d'un substrat nutritif.

13. Procédé de préparation du yaourt, <u>caractérisé</u> en ce qu'on ensemence un produit lacté avec une culture bactérienne mixte selon une des revendications 1 à 12 et on le fait incuber de la manière habituelle.

14. Procédé selon la revendication 13 <u>caractérisé</u> en ce que le produit lacté a été soumis à un traitement par la chaleur usuel avant d'être ensemencé, et a ensuite été refroidi à une température d'environ 44 à 46°C.

15. Procédé selon la revendication 13 et/ou la revendication 14, <u>caractérisé</u> en ce qu'on ensemence le produit lacté avec de 1 à 10 %, de préférence de 3 à 5 %, d'une culture mixte selon l'une des revendications 1 à 12, on l'introduit dans des pots pour la vente, et ensuite on le fait incuber à 42°C.

16. Procédé selon l'une au moins des revendications 13 à 15, <u>caractérisé</u> en ce que, lorsque le pH a atteint une valeur d'environ 4,75, on arrête l'acidification par refroidissement.

17. Procédé selon la revendication 13 et/ou la revendication 14, <u>caractérisé</u> en ce qu'on conserve le produit lacté à une température d'environ 32 à 35°C dans un réservoir de maturation, après quoi on l'ensemence avec d'environ 0,5 à 2 % d'une culture mixte selon au moins une des revendications 1 à 4, on le fait incuber pendant une durée d'environ 12 à 20 h à une température d'environ 32 à 35°C et, lorsque le pH a atteint une valeur d'environ 4,0 à 4,4, on l'introduit dans des récipients pour la vente.

18. Procédé selon l'une au moins des revendications 13 à 17, <u>caractérisé</u> en ce que le produit lacté est constitué par de la crème, par du lait entier, par du lait appauvri en matières grasses ou par du lait maigre.

19. Procédé selon lune au moins des revendications 13 à 18, <u>caractérisé</u> en ce qu'on ajoute une préparation fruitée.